# EUROPEAN PATENT SPECIFICATION

(11) **EP 2 934 325 B1**
(45) Date of publication and mention of the grant of the patent: **13.12.2017**
(21) Application number: 13865226.8
(22) Date of filing: 18.12.2013
(51) Int. Cl.: A61B 8/00, A61B 5/15, A61B 5/00, A61B 5/0215, A61B 5/153, A61M 25/06, A61M 25/00

(54) **INTRODUCER HAVING A FLOW SENSOR**
EINFÜHRVORRICHTUNG MIT EINEM DURCHFLUSSSENSOR
DISPOSITIF INTRODUCTEUR DOTÉ D'UN CAPTEUR D'ÉCOULEMENT

(30) Priority: 21.12.2012 US 201261745394 P
(43) Date of publication of application: 28.10.2015
(73) Proprietor: Volcano Corporation, San Diego, CA 92130 (US); Stigall, Jeremy, San Diego, CA 92130 (US)
(72) Inventor: STIGALL, Jeremy, San Diego, CA 92130 (US)
(74) Representative: de Haan, Poul Erik
(86) International application number: PCT/US2013/076304
(87) International publication number: WO 2014/100286

(56) References cited:
- US-A- 4 274 423
- US-A- 4 887 606
- US-A- 5 480 388
- US-A1- 2009 105 597
- US-A1- 2010 094 143
- US-A1- 2011 066 073
- US-A1- 2012 238 956
- US-A1- 2012 296 275

## Description

### FIELD OF THE INVENTION

The invention relates to needles, catheters, and introducers used to deliver liquids, guide wires, or endovascular devices to the vasculature of a subject. The invention also provides improved methods for inserting a needle into the vasculature of a subject.

### BACKGROUND

When fluids or devices are delivered to the vasculature, a cannula (i.e., tube) is often placed into the vein or artery to provide safer access. The cannula typically is formed of a resilient biocompatible polymer, and has a port (e.g., Luer-Lock™) on the proximal end (exterior) to attach an I.V. tube, or to provide access for devices. The cannula is usually delivered with a metal needle or some other sharp instrument (trocar) designed to move through the skin and allow the cannula to be inserted into the vessel. Some designs deliver the cannula over the needle (trocar), while some designs deliver the cannula within the needle (trocar). Once the cannula is in place, the needle can be removed, thus avoiding complications such as the needle perforating the vessel or other tissue. A number of vascular insertion devices deliver cannulas with this method, including central lines, peripherally inserted central catheter (PICC) lines and introducers (i.e., introducer sheaths), used for delivering devices (e.g., catheters).

Arterial cannula (e.g., lines) can be placed in multiple arteries, including the radial, ulnar, brachial, axillary, dorsalis pedis, posterior tibial, and femoral arteries. The most common site of cannulation is the radial artery, followed by the femoral artery. If the cannula will be in place for a long period of time (e.g., a day), the radial artery is the site of choice due to its ease of cannulation, ease of observation, and low rate of complications. If a larger device will be delivered to the vasculature, the cannula often will be placed in the femoral artery, however cannula in the femoral artery greatly limit a patient's mobility. Femoral lines are primarily used in procedural settings, e.g., catheterization labs.

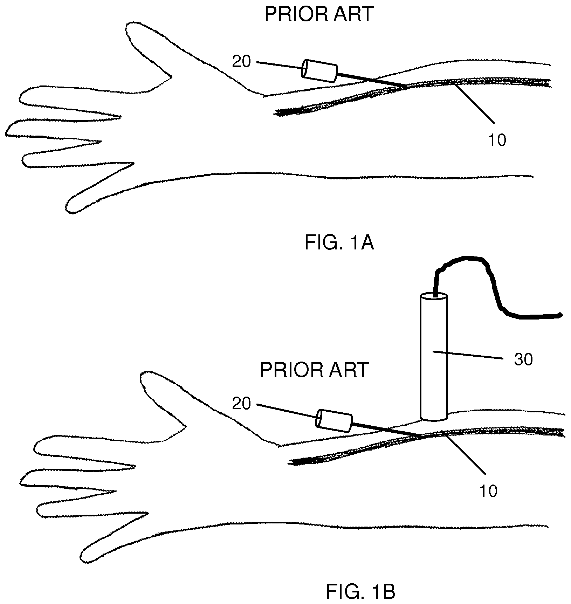

Diagrams showing conventional methods of placing an introducer (including a cannula) into the radial artery are shown in FIG. 1A and 1B. FIG. 1A shows the simplest procedure for entering the radial artery, wherein the radial artery 10 is identified by simply palpating the forearm to locate a pulsatile tubular structure corresponding to the radial artery 10. Once the radial artery 10 is identified, an introducer 20, having a needle is inserted into the radial artery 10 creating a pathway for blood, fluids, devices, etc. While the procedure depicted in FIG. 1A can be done quickly by a trained professional on a healthy patient, the procedure can be challenging if the patient is obese or has bad vasculature. Furthermore, there is a measureable rate of complication from radial inserter misplacement, including nerve damage and arterial damage.

When presented with a challenging patient, a Doppler probe 30 can be used to locate the radial artery 10 for placement of the introducer 20, as depicted in FIG. 1B. The Doppler probe 30 is placed next to the skin and used to identify at least two points on the forearm that have the signature of an artery, i.e., pulsating fluid flow. The points are assumed to roughly follow a line corresponding to the radial artery 10. The introducer 20 is then inserted along this determined line.

External Doppler imaging for placement, i.e., the method depicted in FIG. 1B, suffers from several shortcomings. First, the points identified as corresponding to the radial artery 10 are inexact. Because the Doppler probe 30 is looking through the skin, it is difficult to know exactly where the artery is beneath the skin. In many cases the points are an educated guess as to the location of the radial artery 10. Second, once the artery is "identified" it can be very hard to insert the introducer directly into the radial artery 10. That is, even using the Doppler probe 30, it is possible to miss the artery and damage the nerves or tissues nearby the artery. It is also possible to nick the artery producing blood, but not delivering access. Third, Doppler imaging the forearm to locate the radial artery 10 is time consuming because the Doppler equipment has to be located and prepared prior to delivering the introducer. Additionally, the insertion site often has to be re-prepped after Doppler imaging, i.e., prior to breaking the skin with the introducer. The extra time needed for these tasks can have negative consequences if the patient is in critical need of care.

US 4,887,606 discloses an Apparatus for use in cannulation of blood vessels including a hollow needle having a sharpened end for penetrating tissue, a trocar including a transducer mounted on one end for positioning within the hollow needle for transmitting and receiving ultrasonic waves through the sharpened end of the needle. The trocar further includes a support rod for the transducer and ultrasound damping material attaching the transducer to the support rod.

US 2012/0296275 A1 describes a medical device including a sheath having a proximal end and a distal end, the sheath having an opening at both the proximal end and the distal end, and a removable dilator having a distal end and a proximal end, the dilator having at least one blood detector disposed on a tapered portion of the distal end of the dilator, the dilator positionable within the sheath such that the tapered portion of the distal end of the dilator extends through the opening in the distal end of the sheath, the proximal end of the dilator extends through the opening in the proximal end of the sheath, and at least a portion of the at least one blood detector is adjacent to the distal end of the sheath.

US 2009/0105597 A1 describes a remotely manipulatable ultrasound transducer element or transducer array permiting an operator of an ultrasound system to be remotely located from a patient and yet remotely controlling the location of the element or array on a patient's body such as on the skin surface or within a body cavity. The transducer element or transducer array associated with motors and control circuits comprises an assembly within a housing for fixation to or within a human body. The transducer assembly may be fixed to a ring surrounding an image guided catheter and may rotate about the image guided catheter or move along its length to an anchoring position proximate the surface skin. Two embodiment systems for pericardial access may comprise surface and internal vision or ultrasound guidance systems that are wireless or wired one operating on suction and another on mechanical grasping of the pericardial lining.

### SUMMARY

The shortcomings of prior art introducers are overcome with the disclosed invention as defined by the appended claims. The invention provides a vascular insertion device as defined in claim 1. The design allows a user to identify the target vessel, with the needle, while the needle is being inserted into the body. The device will save time and reduce complications caused by improper placement, such as vascular perforation. Furthermore, a user can easily place a cannula into an artery of a patient with bad vasculature, or with hard-to-identify vasculature (e.g., obese).

Further advantages are achieved by the embodiments indicated by the dependent claims.

In one instance, there is described an introducer incorporating a needle having a flow sensor. In an example, the flow sensor is incorporated into a guide shaft which is inserted into the needle. The guide shaft interfaces with a monitor allowing a user to identify a vein or artery as the introducer is being placed. The introducer will typically include a sheath on the outside of the needle. Once the introducer is placed, the needle can be removed, leaving the sheath in the artery to provide access for additional procedures and devices.

In another instance, there is described a stand-alone flow sensor guide shaft. The guide shaft is different from a guide wire, being of a shorter length, e.g., 30 cm or less, and an outer diameter of 1 mm or less. The guide shaft includes a distal end having an ultrasonic transducer and a proximal end having an electrical connector. In some examples, the guide shaft will also include a pressure sensor. Also described is a method. In one instance, the method includes inserting the insertion tip of an introducer into a subject, monitoring a signal to determine the proximity of a blood vessel, and inserting the introducer into the blood vessel of the subject. When the introducer includes a sheath, i.e. a tube, the needle can be removed from the vessel, leaving the sheath in the artery and providing a cannula for access to the artery.

### BRIEF DESCRIPTION OF THE DRAWINGS

FIG. 1A depicts a prior art method of placing an introducer into a radial artery;
FIG. 1A depicts a prior art method of placing an introducer into a radial artery;
FIG. 2 shows the components of an introducer;
FIG. 3 shows a detail view of the tip of a needle with a guide shaft having a flow sensor within;
FIG. 4 shows a detail view of the tip of a needle with a guide shaft having a flow sensor and a pressure sensor within;
FIG. 5 depicts a distal end of a guide shaft having a flow sensor and comprising a wire;
FIG. 6 depicts a distal end of a guide shaft having a flow sensor and comprising a polymer tube;
FIG. 7 depicts a distal end of a guide shaft having a flow sensor and a pressure sensor;
FIG. 8 depicts a proximal end of a guide shaft having a plurality of electrical connectors;
FIG. 9 depicts placing a radial introducer of the invention with audible signal guidance;
FIG. 10 depicts placing a radial introducer of the invention with visual signal guidance.

### DETAILED DESCRIPTION

The invention discloses improved vascular insertion devices (e.g., introducers) and describes methods for inserting needles (including introducers) into a blood vessel of a subject. Using the devices of the invention, it is safer and easier for a professional to insert a needle into a blood vessel (e.g., a vein or artery). In particular, the disclosed devices detect the flow of blood in a vessel using a sensor at the tip of the needle as the needle is moving into the skin and toward the vessel. The devices additionally include a pressure sensor that provides confirmation that the needle has entered the vessel.

### Introducer

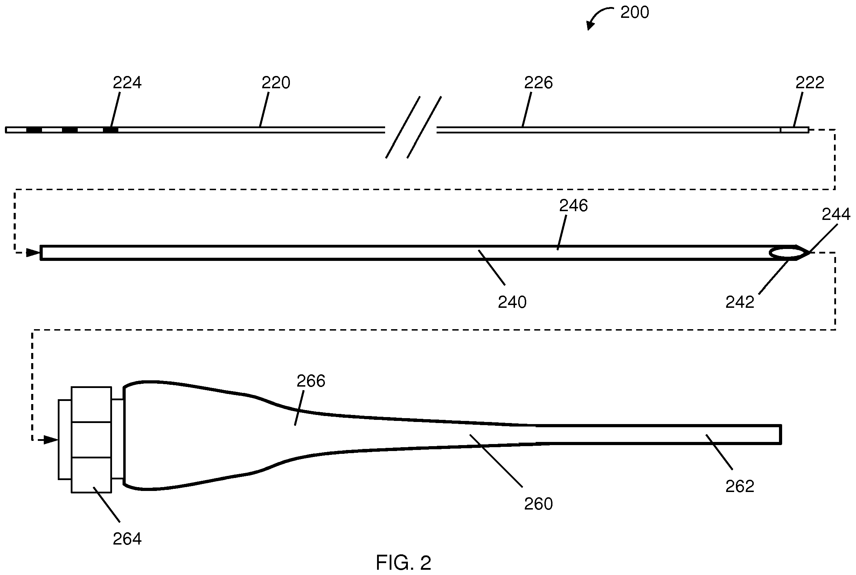

A generalized depiction of an introducer 200 according to the invention is shown in FIG. 2. The introducer 200 includes a guide shaft 220, a needle 240, and a sheath 260. As shown by the dashed arrows, the guide shaft 220 is placed inside the needle 240, and then the needle 240 with the guide shaft 220 placed inside is placed into the sheath 260 to complete the introducer 200. Once assembled, the introducer 200 can be inserted through the skin into a blood vessel, e.g., an artery, with guidance from the sensor 222, as described in greater detail below. Once the introducer 200 has been placed in the artery, the needle 240 and the guide shaft 220 can be withdrawn together, leaving the sheath 260 in place in the artery, and providing safe access to the vasculature.

The guide shaft 220 may be constructed in a variety of ways, as discussed in greater detail below, but has several common components, namely a sensor 222, a connector 224 coupled to the sensor 222, and a guide shaft body 226 connecting the sensor 222 and the connector 224. The guide shaft 220 is elongated, typically having a length longer than 10 cm, e.g., longer than 15 cm, e.g., longer than 20 cm, e.g., longer than 25 cm, e.g., longer than 30 cm, e.g., longer than 35 cm, e.g., longer than 40 cm, e.g., longer than 45 cm, e.g., longer than 50cm, e.g., longer than 100 cm. In some embodiments, the guide shaft 220 is about 25 cm. In some embodiments, the guide shaft 200 is shorter than 50 cm, e.g., shorter than 40 cm, e.g., shorter than 30 cm. The guide shaft 200 is typically 1 mm or smaller in outer diameter, e.g., 1 mm or smaller, e.g., 0.7 mm or smaller, e.g., 0.5 mm or smaller, e.g., 0.4 mm or smaller. In some embodiments, the guide shaft 200 has an outside diameter of 0.46 mm (0.018").

The needle 240 can be any standard delivery needle, or a specially-designed sharp device for inserting the sheath into the skin. The needle 240 will include an opening 242 at a tip 244 and a body 246. The opening 242 is designed to allow the sensor 222 to measure/monitor the environment around the tip 224. Accordingly, while shown as an oval, the opening 242 could also be square, triangular, trapezoidal, etc. The needle is typically constructed from metal, e.g., hypodermic surgical tubing. The needle can be gauge 18 or smaller, for example gauge 19, gauge 20, gauge 21, gauge 22, gauge 23, or gauge 24.

The sheath 260 fits over the needle 240, and will provide access to a blood vessel after placement, e.g., with removal of needle 240 containing guide shaft 220. The sheath comprises distal tube 262, port 264, and body 266. The sheath may be fabricated from any polymer approved for placement into the body having suitable mechanical properties, such as fluoropolymers including perfluoronated ethylene-propylene copolymers (EFEP) and polytetrafluoroethylene (PTFE). Other polymers, such as polyethylene, polyamides, such as polyether block amide copolymers (PEBA), and polyimides can be used to fabricate the sheath. In some embodiments a blend of two or more polymers may be used, created with coextrusion or melt processing. The sheath may also be coated to improve lubricity on the interior and exterior surfaces. Coatings may include, for example, silicone, waxes, or other hydrophobic coatings. Coatings may also include hydrophilic coatings that help provide better wetting of the sheath materials. Functionalized EFEP copolymers are available from commercial sources as NEOFLON™ RP series resins (Daikin America, Inc., Orangeburg, N.Y., USA) and PEBA is available from by Arkema (Colobes Cedex, France).

The port 264 on the sheath 260 can be any common port that is used in a medical setting or a proprietary port. The port may be, for example a Luer-Lock™, an industry standard tapered termination used by most syringe manufacturers including medical tubing and syringes. The port may include a valve (e.g., a hemostasis valve) or a diaphragm to prevent backflow of blood. Alternatively, the port may have a coupling, or other mechanism to secure or anchor the introducer, or to secure or anchor a device introduced through the introducer. In some embodiments, the introducer additionally includes a sideport having a branching tube, a valve, and an additional port for adding or removing fluids through the introducer.

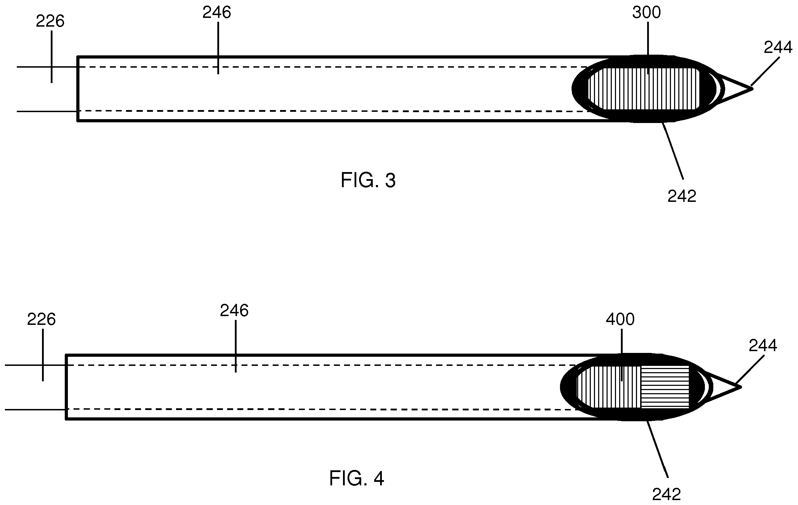

The guide shaft 220 is designed to fit within the needle 240 and provide a clear monitoring field from the tip 244. This concept is exemplified in detail in FIGS. 3 and 4. FIG. 3 shows the body 226 of the guide shaft 200 fitting within the lumen defined by the walls of the body 246 of the needle 240. A flow sensor 300 at the proximal end of the guide shaft 220 emerges from the opening 242, allowing a user to probe a tissue for signatures of blood flow (described in greater detail below). The flow sensor 300 can be of any suitable design. In one embodiment, the flow sensor 300 is an ultrasonic sensor comprising a piezoelectric element (discussed in greater detail below). As shown in FIG. 4, the body 226 of the guide shaft 200 fits within the lumen defined by the walls of the body 246 of the needle 240 and provides a combined flow/pressure sensor 400 at the proximal end of the guide shaft 220. The combined flow/pressure sensor 400 allows a user to probe a tissue for signatures of blood flow and provides an immediate indication that a blood vessel has been entered (described in greater detail below). The combined flow/pressure sensor 400 can be of any suitable design. In one embodiment, combined flow/pressure sensor 400 comprises an ultrasonic sensor and a piezoelectric pressure sensor (discussed in greater detail below).

### Guide shaft

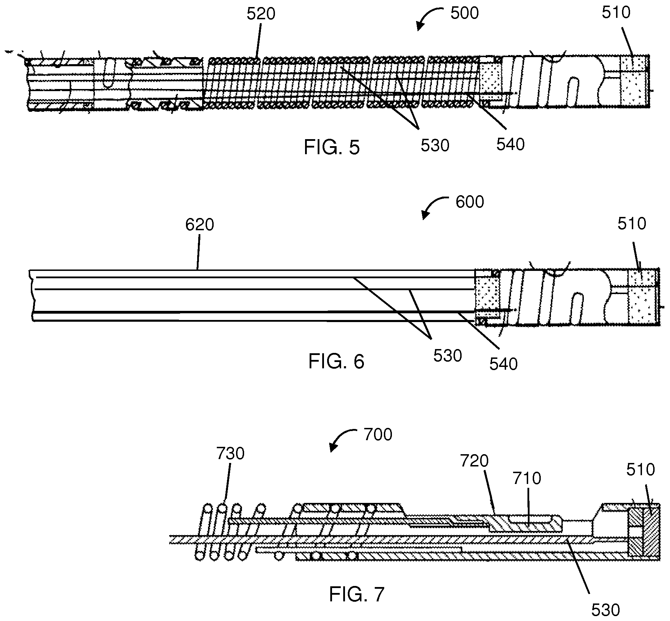

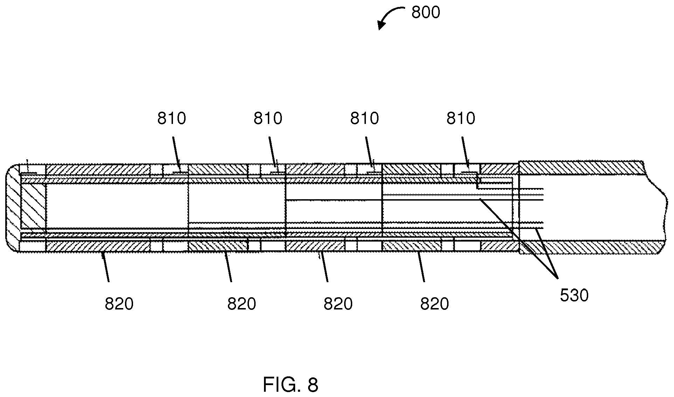

A distal tip of a metal guide shaft 500 with a pressure sensor is exemplified in FIG. 5. The metal guide shaft 500 is comprised of a flexible elongate member 520 that is formed from metal, typically metal coils. The metal is typically surgical steel, stainless steel, or another resilient biocompatible alloy such as nitinol. An ultrasonic transducer 510 is secured to the distal tip using a mechanical connection or epoxy, or some other adhesive. The transducer has electrical leads connected to signal wires 530 that extend the length of the metal guide shaft 500 and terminate with connectors detailed in FIG. 8. In some embodiments, the transducer 510 is open to the environment. In some embodiments the ultrasonic transducer 510 is covered with a polymer layer or some other coating. The metal guide shaft 500 comprises a core wire 540 that provides shapability to the metal guide shaft 500, that is, it allows the user to bend the guide shaft 500 into a specific shape and the shape will be retained.

In some embodiments, the ultrasonic transducer 510 will comprise a piezoelectric element, for example an element formed from a piezoelectric ceramic or crystal. Suitable piezoelectric materials include EC-98 lead magnesium niobate available from EDO Corporation (Salt Lake City, Utah) and PZT-5H from Verniton (Bedford, Ohio). The ultrasonic transducer 510 is not limited to piezoelectric elements, however, as it can be fabricated using a photoacoustic material driven by an optical pulse, e.g., from a pulsed light source, delivered by an optical fiber. Suitable ultrasonic transducers using photoacoustic materials are disclosed in US. Patent Nos. 7,527,594 and 8,059,923.

In an alternative embodiment, the guide shaft is a polymer guide shaft 600, exemplified in FIG. 6. The polymer guide shaft 600 is comprised of a flexible elongate member 620 that is formed from a polymer tube, typically a resilient polymer. The polymer may be polyethylene, a fluoropolymer (such as EFEP or PTFE), or a polyamide, including polyether block amide copolymers (PEBA), or another resilient biocompatible polymer. An ultrasonic transducer 510 is secured to the distal tip using epoxy, or some other adhesive. The ultrasonic transducer 510 has electrical leads connected to signal wires 530 that extend the length of the polymer guide shaft 600 and terminate with connectors detailed in FIG. 8. In some embodiments, the ultrasonic transducer 510 is open to the environment. In some embodiments, the ultrasonic transducer 510 is covered with a polymer layer or some other coating. The polymer guide shaft 600 comprises a core wire 540 that provides shapability to the polymer guide shaft 600, that is, it allows the user to bend the guide shaft 600 into a specific shape and the shape will be retained. Like the metal guide shaft 500, the polymer guide shaft may alternatively use photoacoustic material driven by an optical pulse, e.g., from a pulsed light source, delivered by an optical fiber to make flow measurements. A metal or a plastic guide shaft may additionally comprise a pressure sensor. An exemplary distal end of a dual sensor guide shaft 700 is shown in FIG. 7. (It should be noted that the scale of FIG. 7 is depicted with a greater magnification than the distal tips in FIGS. 5 and 6). The dual sensor guide shaft 700 includes an ultrasonic transducer 510, usable as a Doppler flow sensor, disposed at or in close proximity to the distal end of the dual sensor guide shaft 700. The ultrasound transducer 510 may be any suitable transducer, and may be mounted in the distal end using any conventional method, including the manner described in U.S. Pat. No. 5,125,137. Signal wires 530 may be secured to the front and rear sides of the ultrasound transducer 510 and the signal wires 530 may extend interiorly to the proximal extremity of the dual sensor guide shaft 700. As shown in FIG. 7, dual sensor guide shaft 700 may include a supporting coil body 730, similar to the flexible elongate member 520 discussed with respect to FIG. 5.

The dual sensor guide shaft 700 also includes a pressure sensor 710 also disposed at or in close proximity to the distal end of the dual sensor guide shaft 700. The pressure sensor 710 may be of the type described in U.S. Pat. No. 6,106,476. For example, the pressure sensor 710 may be comprised of a crystal semiconductor material having a recess therein and forming a diaphragm bordered by a rim. A reinforcing member may be bonded to the crystal to reinforce the rim of the crystal, and may have a cavity therein underlying the diaphragm and exposed to the diaphragm. A resistor having opposite ends may be carried by the crystal and may have a portion thereof overlying a portion of the diaphragm. Leads may be connected to opposite ends of the resistor and extend proximally within the dual sensor guide shaft 700. Additional details of suitable pressure sensors that may be used as the pressure sensor 710 are described in U.S. Pat. Nos. 6,106,476. U.S. Pat. No. 6,106,476 also describes suitable methods for mounting the pressure sensor 710 within the dual sensor guide shaft 700. In one embodiment, as shown in FIG. 7, the pressure sensor 710 is oriented in a cantilevered position within a sensor housing 720. For example, the sensor housing 720 preferably includes a lumen surrounded by housing walls. When in a cantilevered position, the pressure sensor 710 projects into the lumen of the sensor housing 720 without contacting the walls of the sensor housing 720.

As depicted in FIG. 7, the dual sensor guide shaft 700 incorporates a sensor housing 720 designed to enclose both the ultrasound transducer 510 and the pressure sensor 710. One advantage of the sensor housing 720 is that because the sensor housing 720 encloses both the ultrasound transducer 510 and the pressure sensor 710, the profile of the sensor package is small and can easily access the surroundings via the opening 242 in the needle.

An embodiment of the proximal end of a guide shaft 800 of the invention is shown in FIG. 8. As shown in FIG. 8, the proximal end 800 comprises connectors 810 and 820 whose function is to provide power and signal to the ultrasonic transducer 510 and/or the pressure sensor. The connectors 810 and 820 comprise contact surfaces that run around the circumference of the proximal end 800. Usually two electrical connectors 810/820 are necessary for a stand-alone flow measurement guide shaft. A guide shaft for a dual sensor guide shaft will require at least five signal wires 530 and at least five electrical connectors 810/820. The connectors 810 and 820 may be electrically isolated from each other by means of a nonconducting material (PTFE) or simply epoxy. Alternatively, polyimide tubes may be used to isolate conductors from the conductive bands.

While not shown in detail here, the proximal end of the guide shaft is easily interfaced with a matching socket, for example a socket described in U.S. Patent No. 8,277,386. A suitable socket has corresponding conductive bands to make connections with electrical connectors 810/820 of the guide shaft to transmit the electrical signals to an instrument, such as, e.g., a signal output or monitor, thereby allowing the flow or pressure measurements to be used to place the introducer. Because the proximal end is easily interfaced with the socket, it is easy to connect the introducer to the needed equipment, place the introducer with assistance of a signal output, and then remove the guide shaft along with the needle to provide cannular access for further procedures.

### Placing the Introducer

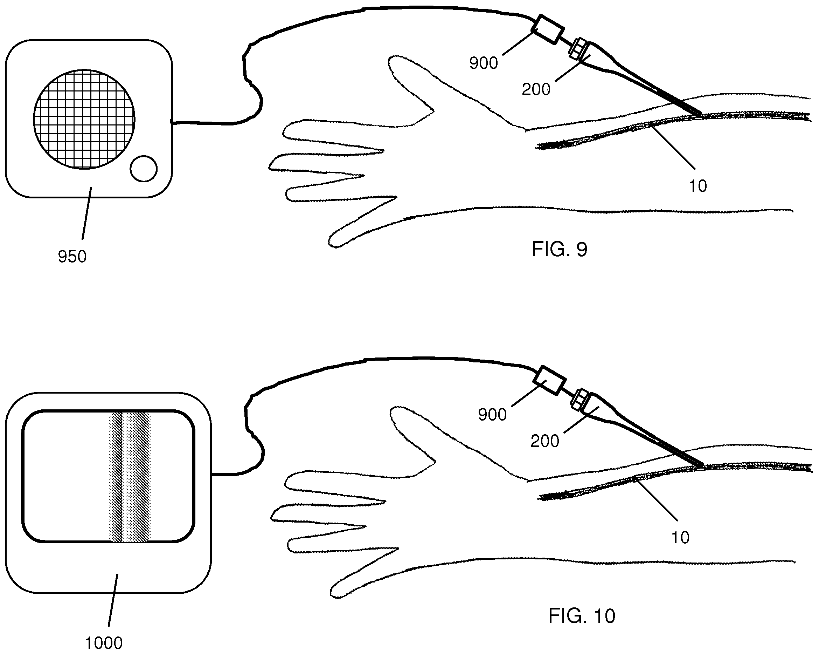

Methods of using the disclosed introducers are exemplified in FIGS. 9 and 10. Both FIG. 9 and FIG. 10 show placement of an introducer 200 into the radial artery, being guided by a signal output. FIG. 9 exemplifies using an audible signal to guide placement, while FIG. 10 exemplifies using a visual indicator. It is understood that a combination of audible and visual placement is possible. The proximal end of the guide shaft, detailed in FIG. 8 is interfaced with socket 900 allowing a connection to either an audible output 950 or a visual display 1000. The signal may be delivered directly to the audible output 950 or a visual display 1000. The signals may be delivered analog or digital. Typically signal conversion is done within the audible output 950 or the visual display 1000 to reduce the heft of the socket 900, making it easier to place introducer 200. Techniques for converting the Doppler signal or pressure reading into an audible or visual output are known.

Prior to placing the introducer 200, the site is prepped according to local practice, which may include washing the area with an antiseptic. Either visually, or with a finger, the approximate location of the radial artery 10 is noted, The sharp tip of the introducer is then inserted at about a 45° angle a short distance into the skin in proximity to the radial artery 10, while monitoring the Doppler signal for indications of nearby blood flow. Using the tip of the introducer as a pivot point, the introducer is rocked and rotated to determine a path toward the radial artery 10. With an audible indicator, the radial artery 10 may sound like a pulsatile wave. With a visible indicator the radial artery 10 will appear as a darker (or lighter) color in a given direction. Once the direction of the radial artery 10 is identified, the introducer is inserted further until a marked change in the audible or visual indicator is detected, corresponding to entering the radial artery 10. At this point the introducer is flattened toward the skin with some wiggling to optimize the Doppler signal within the radial artery 10. The introducer can then be fully inserted into the radial artery 10, the introducer secured, and then the needle and the guide shaft can be removed leaving a cannulated artery. Examples having a pressure sensor will be placed with the same technique; however the pressure sensor offers a few advantages over the Doppler sensor alone. First, the pressure sensor can be used to provide a different tone or visual alert that the artery has been entered. That is, the increase in pressure once the artery is reached is quite obvious, and the spike in pressure can be used to trigger an alert. Second, the pressure sensor can quickly verify if a blood vessel entered into by the introducer is an artery (higher pressure) or a vein (lower pressure).

Additional uses for the disclosed introducer, including placement in other vasculature (e.g., femoral artery) will be evident to one of skill in the art. Introducers of the invention may be sold in sterile packaging with instructions. Introducers of the invention may be sold as part of a system including the introducer and electronics for monitoring the pressure or Doppler signals described above along with a hand-held monitor that provides an audible and/or visual indication of the status of the insertion.

Various modifications of the invention and many further embodiments thereof, in addition to those shown and described herein, will become apparent to those skilled in the art from the full contents of this document. The subject matter herein contains important information, exemplification and guidance that can be adapted to the practice of this invention in its various embodiments.

## Claims

1. A vascular insertion device comprising a needle (240) and a guide shaft (220, 500, 600, 700, 800) located within the lumen defined by the needle, wherein a pressure sensor (400, 710) and a flow sensor (300, 400) are integrated into the guide shaft (220, 500, 600, 700, 800), the pressure sensor and the flow sensor being located at a distal tip of the guide shaft, wherein the guide shaft comprises a flexible elongated member (520, 620) and a core wire (540) providing shapability to the guide shaft (220, 500, 600, 700, 800).

2. The vascular insertion device of claim 1, wherein the device additionally comprises a flexible lumen (260) contacting an exterior of the needle.

3. The vascular insertion device of claim 1, wherein the needle (240) is 18 gauge or smaller.

4. The vascular insertion device of claim 1, wherein the flow sensor comprises an ultrasonic transducer (510).

5. The vascular insertion device of claim 1, wherein the pressure sensor (400, 710) comprises a piezoelectric sensor.

6. The vascular insertion device of claim 1, wherein the device comprises a port (264).

7. The vascular insertion device of claim 6, wherein the port (264) is a Luer-type port.

8. The vascular insertion device of claim 1, wherein the guide shaft (220, 500, 600, 700, 800) has an outer diameter of 1 mm or smaller.

9. The vascular insertion device of claim 1, wherein the guide shaft (220, 500, 600, 700, 800) comprises a polymer.

10. The vascular insertion device of claim 1, further comprising a radiopaque label.

11. The vascular insertion device of claim 1, wherein the guide shaft (220, 500, 600, 700, 800) is 30 cm or less in length.

12. The vascular insertion device of claim 4, wherein the ultrasonic transducer (510) produces acoustic waves at a frequency of 5MHz to 15MHz.

13. The vascular insertion device of claim 4, wherein the ultrasonic transducer (510) detects reflected acoustic waves at a frequency of 5MHz to 15MHz.

## Patentansprüche

1. Gefäßeinführvorrichtung, umfassend eine Kanüle (240) und eine Führungswelle (220, 500, 600, 700, 800), die sich innerhalb des von der Kanüle definierten Lumens befindet, wobei ein Drucksensor (400, 710) und ein Durchflusssensor (300, 400) in die Führungswelle (220, 500, 600, 700, 800) integriert sind, wobei sich der Drucksensor und der Durchflusssensor an einer distalen Spitze der Führungswelle befinden, wobei die Führungswelle ein flexibles längliches Bauteil (520, 620) und einen Kerndraht (540) umfasst, der der Führungswelle (220, 500, 600, 700, 800) Formbarkeit verleiht.

2. Gefäßeinführvorrichtung nach Anspruch 1, wobei die Vorrichtung zusätzlich ein flexibles Lumen (260) umfasst, das eine Außenseite der Kanüle berührt.

3. Gefäßeinführvorrichtung nach Anspruch 1, wobei die Kanüle (240) 18 Gauge oder kleiner ist.

4. Gefäßeinführvorrichtung nach Anspruch 1, wobei der Durchflusssensor einen Ultraschallwandler (510) umfasst.

5. Gefäßeinführvorrichtung nach Anspruch 1, wobei der Drucksensor (400, 710) einen piezoelektrischen Sensor umfasst.

6. Gefäßeinführvorrichtung nach Anspruch 1, wobei die Vorrichtung einen Anschluss (264) umfasst.

7. Gefäßeinführvorrichtung nach Anspruch 6, wobei der Anschluss (264) ein Anschluss vom Luer-Typ ist.

8. Gefäßeinführvorrichtung nach Anspruch 1, wobei die Führungswelle (220, 500, 600, 700, 800) einen Außendurchmesser von 1 mm oder kleiner besitzt.

9. Gefäßeinführvorrichtung nach Anspruch 1, wobei die Führungswelle (220, 500, 600, 700, 800) ein Polymer umfasst.

10. Gefäßeinführvorrichtung nach Anspruch 1, weiter eine strahlenundurchlässige Markierung umfassend.

11. Gefäßeinführvorrichtung nach Anspruch 1, wobei die Führungswelle (220, 500, 600, 700, 800) eine Länge von 30 cm oder weniger besitzt.

12. Gefäßeinführvorrichtung nach Anspruch 4, wobei der Ultraschallwandler (510) Schallwellen mit einer Frequenz von 5 MHz bis 15 MHz erzeugt.

13. Gefäßeinführvorrichtung nach Anspruch 4, wobei der Ultraschallwandler (510) reflektierte Schallwellen mit einer Frequenz von 5 MHz bis 15 MHz erkennt.

## Revendications

1. Dispositif d'insertion vasculaire comprenant une aiguille (240) et une tige de guidage (220, 500, 600, 700, 800) située dans le lumen défini par l'aiguille, dans lequel un capteur de pression (400, 710) et un capteur d'écoulement (300, 400) sont intégrés dans la tige de guidage (220, 500, 600, 700, 800), le capteur de pression et le capteur d'écoulement étant situés sur une extrémité distale de la tige de guidage, dans lequel la tige de guidage comprend un élément allongé flexible (520, 620) et un fil central (540) fournissant une malléabilité à la tige de guidage (220, 500, 600, 700, 800).

2. Dispositif d'insertion vasculaire selon la revendication 1, dans lequel le dispositif comprend en outre un lumen flexible (260) touchant un extérieur de l'aiguille.

3. Dispositif d'insertion vasculaire selon la revendication 1, dans lequel l'aiguille (240) est de calibre 18 ou moins.

4. Dispositif d'insertion vasculaire selon la revendication 1, dans lequel le capteur d'écoulement comprend un transducteur à ultrasons (510).

5. Dispositif d'insertion vasculaire selon la revendication 1, dans lequel le capteur de pression (400, 710) comprend un capteur piézoélectrique.

6. Dispositif d'insertion vasculaire selon la revendication 1, dans lequel le dispositif comprend un orifice (264).

7. Dispositif d'insertion vasculaire selon la revendication 6, dans lequel l'orifice (264) est un orifice de type luer.

8. Dispositif d'insertion vasculaire selon la revendication 1, dans lequel la tige de guidage (220, 500, 600, 700, 800) présente un diamètre extérieur de 1 mm ou moins.

9. Dispositif d'insertion vasculaire selon la revendication 1, dans lequel la tige de guidage (220, 500, 600, 700, 800) comprend un polymère.

10. Dispositif d'insertion vasculaire selon la revendication 1, comprenant en outre une étiquette radio-opaque.

11. Dispositif d'insertion vasculaire selon la revendication 1, dans lequel la tige de guidage (220, 500, 600, 700, 800) présente une longueur de 30 cm ou moins.

12. Dispositif d'insertion vasculaire selon la revendication 4, dans lequel le transducteur à ultrasons (510) produit des ondes acoustiques à une fréquence de 5 MHz à 15 MHz.

13. Dispositif d'insertion vasculaire selon la revendication 4, dans lequel le transducteur à ultrasons (510) détecte des ondes acoustiques réfléchies à une fréquence de 5 MHz à 15 MHz.
